# FASCICULE DE BREVET EUROPEEN

(11) **EP 0 820 730 B1**
(45) Date de publication et mention de la délivrance du brevet: **02.01.2002**
(21) Numéro de dépôt: 97401747.7
(22) Date de dépôt: 21.07.1997
(51) Int. Cl.: A61B 17/70

(54) **Plaque d'ostéosynthèse de rachis**
Osteosyntheseplatte für die Wirbelsäure
Spinal osteosynthesis plate

(30) Priorité: 22.07.1996 FR 9609164
(43) Date de publication de la demande: 28.01.1998
(73) Titulaire: Euros, 13600 La Ciotat (FR)
(72) Inventeur: Tisserand, Philippe, 66000 Perpignan (FR)
(74) Mandataire: CABINET BONNET-THIRION

(56) Documents cités:
- EP-A- 0 266 146
- EP-A- 0 739 610
- WO-A-96/16608
- DE-U- 9 417 019

## Description

La présente invention concerne une plaque d'ostéosynthèse de longueur L déterminée, destinée à être positionnée sur un côté de l'épine dorsale et fixée sur des vertèbres en vue de leur solidarisation.

Dans la chirurgie du rachis, il est de pratique courante de solidariser ensemble plusieurs vertèbres endommagées. Cette solidarisation peut être réalisée à l'aide de plusieurs systèmes de solidarisation tels que les systèmes à tiges ou encore les systèmes à plaques.

L'invention porte sur les systèmes de solidarisation à plaques qui, par rapport aux systèmes de solidarisation à tiges, présentent comme avantages, une plus grande rapidité de montage, une possibilité de modifier l'implantation ou de retirer une vis pédiculaire sans démonter le montage des plaques et surtout un encombrement minimum en épaisseur ce qui est toujours souhaitable pour les muscles para-vertébraux.

Toutefois, la solidarisation de vertèbres à l'aide de plaques d'ostéosynthèse de type connu présente un certain nombre d'inconvénients.

En effet, la fixation de plaques d'ostéosynthèse sur les vertèbres entraîne un blocage du segment correspondant de la colonne vertébrale. De ce fait, les segments de la colonne vertébrale adjacents au segment bloqué subissent de fortes sollicitations mécaniques ce qui peut entraîner leur déstabilisation. De plus, la fixation rigide de plaques d'ostéosynthèse sur les vertèbres provoque également une sollicitation au niveau des ancrages des moyens de fixation desdites plaques sur les vertèbres.

Il y a alors un risque de pseudarthrose et des douleurs peuvent apparaître à cause de cette fixation trop rigide ou de contraintes trop importantes spécialement lorsque le segment rigidifié se situe au niveau du sacrum. Il y a aussi un risque d'arrachement des moyens de fixation de la plaque d'ostéosynthèse trop sollicités.

On connaît du document WO-9616608 une tige vertébrale contenant une première partie lombo-sacrée rigide dans toutes les directions, et une seconde partie dorsale qui est rigide dans un plan frontal afin de prévenir des scolioses et plus flexible dans un plan sagittale.

La présente invention propose une nouvelle plaque d'ostéosynthèse perfectionnée qui permet de pallier aux inconvénients précités des plaques d'ostéosynthèses déjà connues, qui est conformée de sorte qu'elle présente une flexibilité qui varie le long de sa longueur L, et qui est caractérisée en ce qu'elle comporte une épaisseur d décroissante d'une extrémité à l'autre, et en ce qu'elle comprend une pluralité de perçages traversants répartis sur ladite longueur L et destinés à recevoir des vis de fixation sur les vertèbres, l'épaisseur dₘᵢₙ minimale de ladite plaque d'ostéosynthèse dépendant de la grosseur des têtes de vis de fixation sur les vertèbres et par là même des dimensions des portions d'entrée desdits perçages apte à accueillir lesdites têtes de vis de fixation.

Ainsi, la plaque d'ostéosynthèse conforme à la présente invention, confère par sa flexibilité, au segment vertébral solidarisé une certaine souplesse qui permet d'atténuer considérablement les contraintes subies par les vertèbres et surtout les disques intervertébraux adjacents lors des mouvements complexes de la colonne vertébrale. La flexibilité de ladite plaque permet en outre de diminuer de façon notable les sollicitations sur les moyens de fixation de celle-ci sur les vertèbres notamment au niveau le plus bas de ladite plaque.

Avantageusement, la plaque d'ostéosynthèse conforme à l'invention présente une flexibilité qui est croissante d'une extrémité à l'autre.

La description qui va suivre en regard des dessins annexés, donnés à titre d'exemples non limitatifs, fera bien comprendre en quoi consiste l'invention et comment elle peut être réalisée.
Sur les dessins annexés :
- la figure 1 est une vue schématique en perspective d'une plaque d'ostéosynthèse conforme à la présente invention,
- la figure 2 est une vue de côté de la plaque représentée sur la figure 1.

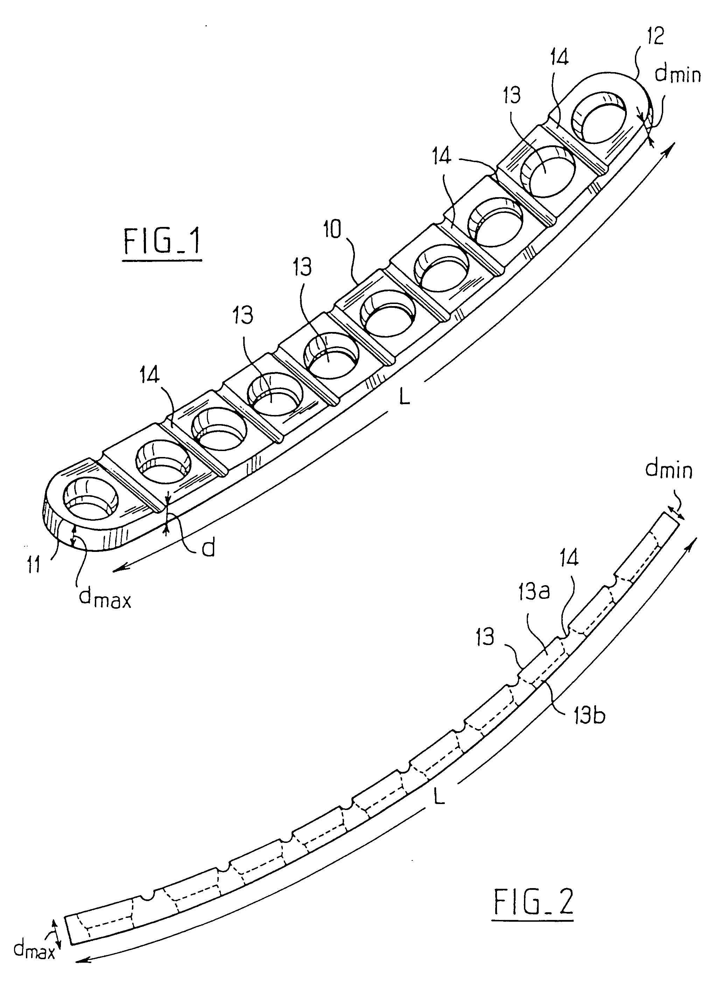

Sur les figures 1 et 2 , on a représenté une plaque d'ostéosynthèse 10 qui fait partie d'un système de solidarisation de plusieurs vertèbres endommagées. Un tel système de solidarisation comprend au moins deux plaques d'ostéosynthèse telles que représentées, ces plaques étant destinées à être positionnées de part et d'autre de l'épine dorsale et fixées sur les pédicules desdites vertèbres.

Comme on peut le voir sur les figures, la plaque d'ostéosynthèse 10 présente une longueur L déterminée. Elle possède une certaine cambrure qui épouse ici la forme anatomique du rachis lombaire.

La plaque d'ostéosynthèse 10 comporte des perçages 13 régulièrement répartis sur toute la longueur L entre ses deux extrémités 11, 12. Dans le mode de réalisation représenté, tous les perçages 13 sont identiques. Ils comportent chacun une portion d'entrée 13a de forme tronconique destinée à recevoir une tête de vis de fixation. En outre, cette portion d'entrée tronconique 13a est prolongée par une portion cylindrique recevant le corps de la vis de fixation.

La plaque d'ostéosynthèse 10 est donc fixée par vissage sur les pédicules des vertèbres endommagées à solidariser.

Il est à noter que les vis de fixation des plaques d'ostéosynthèse sur les vertèbres comportent des têtes normalisées. Il s'en suit qu'en ce qui concerne les perçages dans la plaque d'ostéosynthèse, même si le corps cylindrique 13b des perçages traversant 13 peut présenter des dimensions variables, la portion d'entrée 13a destinée à accueillir la tête de la vis présente des dimensions adaptées aux têtes de vis normalisées et ne peuvent donc varier.

La plaque d'ostéosynthèse représentée sur les figures 1 et 2 présente comme caractéristique originale, une flexibilité qui varie le long de sa longueur L. Plus particulièrement, cette flexibilité est croissante d'une extrémité à l'autre de ladite plaque. La flexibilité de la plaque est ici donnée par le fait que l'épaisseur d de la plaque est décroissante d'une extrémité 11 où l'épaisseur est maximum dₘₐₓ à l'autre extrémité 12 où elle est minimum dₘᵢₙ.

L'épaisseur d de la plaque d'ostéosynthèse représentée comporte une valeur minimale dₘᵢₙ qui dépend de la grosseur des têtes de vis de fixation et donc des dimensions de la portion d'entrée 13a des perçages 13 prévus dans ladite plaque. L'épaisseur minimum étant fixée par la grosseur des têtes normalisées des vis de fixation, il est parfois nécessaire d'augmenter la flexibilité de ladite plaque d'ostéosynthèse. C'est pour cela que comme le montre les figures 1 et 2, il est prévu également sur une face de la plaque d'ostéosynthèse 10 des encoches transversales 14 qui s'étendent sur toute la largeur de la plaque d'ostéosynthèse et sont ici régulièrement répartis le long de la longueur L de la plaque d'ostéosynthèse 10. Chaque encoche 14 est positionnée entre deux perçages 13 successifs. Ces encoches 14 permettent d'augmenter la souplesse de la plaque d'ostéosynthèse sans entamer les lamages fonctionnels pour vis de fixation.

La plaque d'ostéosynthèse 10 représentée est une plaque d'ostéosynthèse lombo-sacré. Elle se positionne de telle sorte que la partie la plus épaisse est fixée sur le sacrum et plus l'on monte sur le long de la colonne vertébrale puis la plaque s'affine jusqu'à arriver à une certaine épaisseur dₘᵢₙ.

Une telle plaque confère alors au segment solidarisé une souplesse permettant de soulager les segments adjacents de la colonne vertébrale. En particulier, les vertèbres et les disques intervertébraux sus-jacents sont ainsi soulagés. Une telle plaque d'ostéosynthèse permet d'une part de diminuer les risques de déstabilisation des segments adjacents au segment rigidifié et d'autre part de diminuer les contraintes exercées sur la vis de fixation fixée sur le sacrum, ces contraintes étant souvent à l'origine de douleurs.

La plaque d'ostéosynthèse 10 conforme à la présente invention, est réalisée par exemple en titane.

Il est intéressant de préciser pour solidariser un petit nombre de vertèbres, en général deux vertèbres, la plaque d'ostéosynthèse selon l'invention présente une longueur L insuffisante pour permettre une variation de son épaisseur d en vue de lui conférer une certaine flexibilité. Dans ce cas, la flexibilité variable de ladite plaque d'ostéosynthèse est conférée uniquement par les encoches transversales réparties sur la longueur L de la plaque et intercalées entre les perçages de celle-ci.

La présente invention n'est nullement limitée aux modes de réalisation décrits et représentés, mais l'homme du métier saura y apporter toutes variantes conformes à son esprit.

En particulier, on peut envisager que la plaque d'ostéosynthèse selon l'invention soit formée d'une succession d'éléments de plaques accolés suivant la longueur, chaque élément de plaque présentant une flexibilité constante différente de sorte que la mise bout à bout desdits éléments constitue une plaque dont la flexibilité varie et en particulier est croissante d'une extrémité à l'autre de la plaque.

## Revendications

1. Plaque d'ostéosynthèse (10) de longueur L déterminée, destinée à être positionnée sur un côté de l'épine dorsale et fixée sur des vertèbres en vue de leur solidarisation, ladite plaque d'ostéosynthèse (10) étant conformée de sorte qu'elle présente une flexibilité qui varie le long de sa longueur L, **caractérisée en ce qu'**elle comporte une épaisseur d décroissante d'une extrémité (11) à l'autre (12), et **en ce qu'**elle comprend une pluralité de perçages traversants (13) répartis sur ladite longueur L et destinés à recevoir des vis de fixation sur les vertèbres, l'épaisseur dₘᵢₙ minimale de ladite plaque d'ostéosynthèse (10) dépendant de la grosseur des têtes de vis de fixation sur les vertèbres et par là même des dimensions des portions d'entrée desdits perçages (13) apte à accueillir lesdites têtes de vis de fixation.

2. Plaque d'ostéosynthèse selon la revendication 1, **caractérisée en ce que** sa flexibilité est croissante d'une extrémité (11) à l'autre (12).

3. Plaque d'ostéosynthèse selon l'une des revendications 1 ou 2, **caractérisé en ce qu'**elle comprend une pluralité d'encoches transversales (14) réparties sur sa longueur L.

4. Plaque d'ostéosynthèse selon l'une quelconque des revendications 1 à 3, **caractérisée en ce qu'**elle présente une cambrure qui épouse la forme anatomique du rachis lombaire.

5. Plaque d'ostéosynthèse selon la revendication 4, **caractérisée en ce qu'**elle présente une épaisseur maximum dₘₐₓ à son extrémité (11) fixée sur le sacrum et une épaisseur minimum dₘᵢₙ à l'extrémité opposée (12).

## Claims

1. An osteosynthesis plate (10) of particular length L intended to be placed on one side of the dorsal spine and fixed to vertebrae in order to fasten them together, said osteosynthesis plate (10) being conformed so that it has a flexibility that varies along its length L, **characterized in that** it has a thickness d decreasing from one end (11) to the other end (12) and **in that** it includes a plurality of holes (13) passing through it, distributed over said length L, and intended to receive screws for fixing it to vertebrae, the minimum thickness dₘᵢₙ of said osteosynthesis plate (10) depending on the size of the heads of the screws for fixing it to vertebrae and thereby on the dimensions of the entry portions of said holes (13) adapted to accommodate said fixing screw heads.

2. An osteosynthesis plate according to claim 1, **characterized in that** its flexibility increases from one end (11) to the other end (12).

3. An osteosynthesis plate according to either claim 1 or claim 2, **characterized in that** it includes a plurality of transverse notches (14) distributed over its length L.

4. An osteosynthesis plate according to any of claims 1 to 3, **characterized in that** it has a curvature that espouses the anatomical shape of the lumbar spine.

5. An osteosynthesis plate according to claim 4, **characterized in that** it has a maximum thickness dₘₐₓ at its end (11) fixed to the sacrum and a minimum thickness dₘᵢₙ at the opposite end (12).

## Patentansprüche

1. Osteosyntheseplatte (10) mit einer festgelegten Länge L zur Anordnung auf einer Seite einer Wirbelsäule und zur Befestigung an den Wirbeln zu deren Stabilisierung, wobei die Osteosyntheseplatte (10) derart angepasst ist, dass sie eine entlang ihrer Länge L variierende Flexibilität aufweist, **dadurch gekennzeichnet**, das sie eine Dicke d besitzt, die von einem Ende (11) zum anderen (12) abnimmt, und dass sie eine Vielzahl von Durchgangsbohrungen (13) umfasst, die über die Länge L verteilt sind und dazu dienen, Wirbelbefestigungsschrauben aufzunehmen, wobei die minimale Dicke dₘᵢₙ der Osteosyntheseplatte (10) von der Größe der Köpfe der Wirbelbefestigungsschrauben und dadurch von den Abmessungen der für die Aufnahme der Köpfe der Befestigungsschrauben ausgebildeten Eintrittsabschnitte dieser Bohrungen (13) abhängt.

2. Osteosyntheseplatte nach Anspruch 1, **dadurch gekennzeichnet, dass** ihre Flexibilität von einem Ende (11) zum anderen (12) zunimmt.

3. Osteosyntheseplatte nach Anspruch 1 oder 2, **dadurch gekennzeichnet, dass** sie eine Vielzahl von quer verlaufenden Einkerbungen (14) umfasst, die über ihre Länge L verteilt sind.

4. Osteosyntheseplatte nach einem der Ansprüche 1 bis 3, **dadurch gekennzeichnet, dass** sie eine Krümmung aufweist, die die anatomische Form der Lendenwirbelsäule annimmt.

5. Osteosyntheseplatte nach Anspruch 4, **dadurch gekennzeichnet, dass** sie eine maximale Dicke dₘₐₓ an ihrem am Kreuzbein befestigten Ende (11) und eine minimale Dicke dₘᵢₙ am entgegengesetzten Ende (12) aufweist.
